# EUROPEAN PATENT APPLICATION

(11) **EP 4 428 265 A2**
(43) Date of publication of application: **11.09.2024**
(21) Application number: 24153380.1
(22) Date of filing: 23.01.2024
(51) Int. Cl.: C25B 1/04, B01D 53/00, C07C 29/151, C10G 2/00, C25B 1/23, C25B 15/02, C25B 15/08, H01M 8/00

(54) **CARBON DIOXIDE CONVERSION APPARATUS**

(30) Priority: 07.03.2023 JP 2023034960
(71) Applicant: Toshiba Energy Systems & Solutions Corporation, Saiwai-ku Kawasaki-shi Kanagawa (JP)
(72) Inventor: OGAWA, Takashi, Minato-ku, Tokyo (JP)
(74) Representative: Hoffmann Eitle

(57) **Abstract**

A carbon dioxide conversion apparatus 1 includes: a carbon dioxide electrolysis part 3 that includes a cathode chamber 11 to produce a first gas containing carbon monoxide and hydrogen and an anode chamber 12 to produce a second gas containing oxygen and carbon dioxide; a direct-current power supply 14; an organic-substance synthesis part 4 to synthesize an organic substance from a feed gas containing the first gas; a partial oxidation part 5 that includes an oxygen removal chamber 22 to receive the second gas and a partial oxidation chamber 21 to partially oxidize a synthesis residual gas from the organic-substance synthesis part 4 to produce a third gas containing carbon monoxide and hydrogen; and a first measurement control part 33 to control an amount of the second gas from the anode chamber 12 to the oxygen removal chamber 22 to increase an amount of carbon monoxide in the third gas.

## Description

### FIELD

Embodiments relate to a carbon dioxide conversion apparatus and a carbon dioxide conversion method.

### BACKGROUND

Fossil fuels such as natural gas, coal, and petroleum is combusted to generate carbon dioxide (CO₂) which is considered the main factor behind global warming due to a greenhouse effect, and thus the fossil fuels should be used less. Known example methods of reducing the carbon dioxide are performed by removing carbon dioxide from an exhaust gas from a carbon dioxide generation source to reduce an amount of the carbon dioxide emitted to the air and then chemically synthesizing a product from the carbon dioxide removed from the exhaust gas. An example method of the know example methods is performed by reducing carbon dioxide to produce carbon monoxide (CO), and synthesizing an organic substance from the produced carbon monoxide (CO) and hydrogen (H₂), and the example method has developed. A part of a purge gas after producing the carbon monoxide is supplied to a carbon dioxide electrolysis device, and the remainder thereof is rendered harmless by air combustion to be emitted into the air. The carbon dioxide supplied to the carbon dioxide electrolysis device, which includes the carbon dioxide supplied for purging oxygen generated from the carbon dioxide electrolysis device and the carbon dioxide discharged from a combustion apparatus, is emitted to the air without being efficiently used. This inefficient use of the carbon dioxide in the carbon dioxide electrolysis device, demands to develop a carbon dioxide conversion apparatus and a carbon dioxide conversion method to allow the efficient use of the supplied carbon dioxide, the reduction in emission of the carbon dioxide into the air, and efficient production of the carbon monoxide of a reduction product of carbon dioxide.

### BRIEF DESCRIPTION OF THE DRAWINGS

FIG. 1 is a diagram illustrating a carbon dioxide conversion apparatus of an embodiment.
FIG. 2 is a flowchart of controlling a carbon monoxide amount in a third gas in the carbon dioxide conversion apparatus illustrated in FIG. 1.
FIG. 3 is a flowchart of controlling a carbon monoxide/hydrogen ratio in the third gas in the carbon dioxide conversion apparatus illustrated in FIG. 1.
FIG. 4 is a flowchart of controlling a flow rate of a first gas in the carbon dioxide conversion apparatus illustrated in FIG. 1.
FIG. 5 is a flowchart of controlling a flow rate of a carbon dioxide containing gas in the carbon dioxide conversion apparatus illustrated in FIG. 1.

### DETAILED DESCRIPTION

Hereinafter, a carbon dioxide conversion apparatus of an embodiment will be described with reference to the drawings. In each embodiment presented below, substantially the same constituent parts are denoted by the same reference signs, and a description thereof may be partially omitted. The drawings are schematic, and the relation of the thickness and planar dimension, a thickness ratio among the parts, and so on may be different from actual ones. Note that a symbol of "~" in the following description indicates a range between an upper limit value and a lower limit value of respective numerical values. In this case, the range of the numerical values includes the upper limit value and the lower limit value.

A carbon dioxide conversion apparatus of an embodiment includes:
a carbon dioxide capture part configured to capture a carbon dioxide from a carbon dioxide containing gas; a carbon dioxide electrolysis part having a cathode chamber configured to receive the carbon dioxide from the carbon dioxide capture part, and configured to reduce at least one selected from the group consisting of the carbon dioxide and water and produce a first gas containing at least one selected from the group consisting of carbon monoxide and hydrogen, and an anode chamber configured to oxidize an oxidizable substance and produce a second gas containing oxygen and carbon dioxide; a direct-current power supply configured to supply a direct current to the carbon dioxide electrolysis part; an organic-substance synthesis part configured to receive the first gas from the cathode chamber, and configured to synthesize an organic substance from a feed gas containing the first gas; a partial oxidation part having an oxygen removal chamber configured to receive the second gas from the anode chamber, and configured to ionize oxygen in the second gas to produce an oxygen ion a partial oxidation chamber configured to: receive a synthesis residual gas to be discharged from the organic-substance synthesis part, the synthesis residual gas containing the organic substance; partially oxidize the synthesis residual gas using the oxygen ion to be produced in the oxygen removal chamber and produce a third gas containing carbon monoxide and hydrogen; and supply the third gas as a part of the feed gas to the organic-substance synthesis part, and an oxygen permeation membrane provided between the oxygen removal chamber and the partial oxidation chamber; and a first control part configured to control an amount of the second gas to be supplied from the anode chamber to the oxygen removal chamber to increase an amount of carbon monoxide in the third gas to be produced in the partial oxidation chamber.

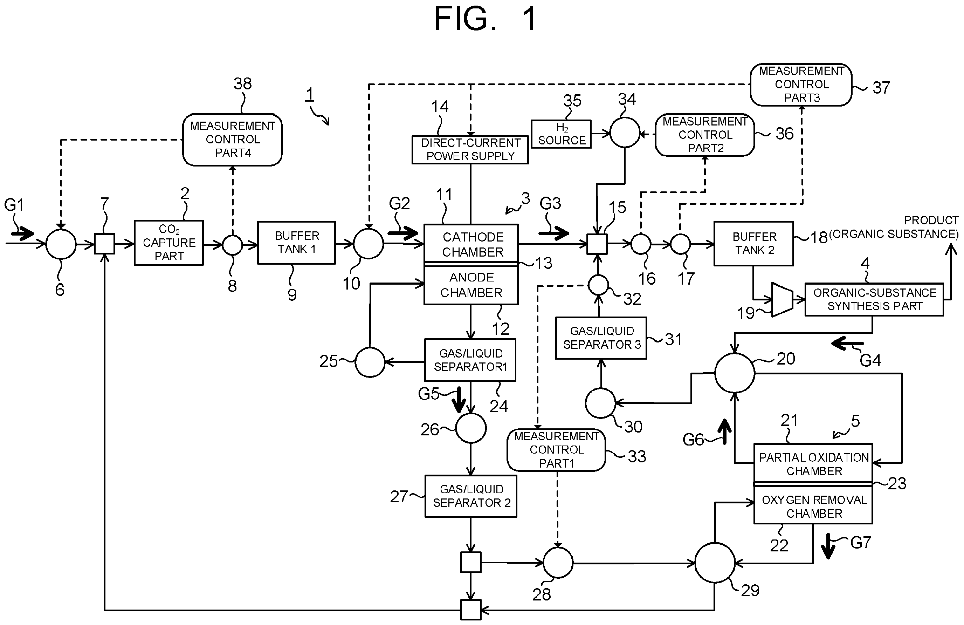

FIG. 1 is a diagram illustrating the carbon dioxide conversion apparatus of the embodiment. A carbon dioxide conversion apparatus 1 illustrated in FIG. 1 includes a CO₂ capture part 2 which captures CO₂ from a carbon dioxide (CO₂) containing gas, a CO₂ electrolysis part 3 which converts CO₂ into carbon monoxide (CO) through electrolysis and reduction, an organic-substance synthesis part 4 which synthesizes an organic substance by using a gas containing the carbon monoxide (CO) and hydrogen (H₂) supplied from the CO₂ electrolysis part 3 as a feed gas, and an oxygen permeation-membrane partial oxidation part 5 which partially oxidizes a synthesis residual gas of the organic substance discharged from the organic-substance synthesis part 4 to produce CO and H₂.

The CO₂ capture part 2 is configured to separate and capture CO₂ from an emission gas G1 containing CO₂ (CO₂ containing gas) emitted from a thermal power plant, a waste incineration plant, a steel plant, and the like, and supply a CO₂ gas G2 having an increased CO₂ concentration to the CO₂ electrolysis part 3. The CO₂ capture part 2 can be constituted by applying, for example, a chemical absorption method of using a chemical absorption solution such as an amine aqueous solution, a physical absorption method of using a physical absorption solution such as methanol or a polyethylene glycol solution, a solid absorption method of using a solid absorbent such as an amine compound, a membrane separation method of using a CO₂ separation membrane, a physical adsorption method of using an inorganic substance such as zeolite as an adsorbent, a PSA (Pressure Swing Adsorption method), a TSA (Thermal Swing Adsorption method), or the like. For example, the chemical absorption method and a chemical absorber which use the amine aqueous solution can supply the emission gas G1 to an absorption tower in which the amine aqueous solution is sprayed, and heat the amine aqueous solution containing the absorbed CO₂ in a regeneration tower to capture CO₂ emitted from the amine aqueous solution. The capture method and device of CO₂ applied to the CO₂ capture part 2 are not particularly limited, and various methods and devices which allow the capture of CO₂ from the emission gas G1 can be applied.

The CO₂ capture part 2 can receive the CO₂ containing gas G1 via a first flow rate regulating valve 6 and a gas mixer 7. The CO₂ capture part 2 is connected to a cathode chamber (reducing part) 11 of the CO₂ electrolysis part 3 via a first flowmeter 8, a first buffer tank 9, and a second flow rate regulating valve 10. The CO₂ capture part 2 is configured to supply a CO₂ gas G2 to the cathode chamber 11 via these components (8, 9, 10).

The CO₂ electrolysis part 3 is a CO₂ electrolysis device having an electrolysis cell, and includes the cathode chamber (reducing part) 11 and an anode chamber (oxidizing part) 12. The cathode chamber 11 includes a reduction electrode (cathode), the anode chamber 12 includes an oxidation electrode (anode), and an electrolytic solution is made to flow through at least the anode chamber 12. A CO₂ gas is made to flow through the cathode chamber 11. Examples of the electrolytic solution supplied into the anode chamber 12, include a solution using water (H₂O), and an aqueous solution containing an optional electrolyte. Examples of the aqueous solution containing the electrolyte, include an aqueous solution containing at least one ion selected from a phosphate ion (PO₄²⁻), a borate ion (BO₃³⁻), a sodium ion (Na⁺), a potassium ion (K⁺), a calcium ion (Ca²⁺), a lithium ion (Li⁺), a cesium ion (Cs⁺), a magnesium ion (Mg²⁺), a chloride ion (Cl⁻), a hydrogen carbonate ion (HCO₃⁻), a carbonate ion (CO₃²⁻), and a hydroxide ion (OH⁻). Examples of the electrolytic solution include an alkaline aqueous solution containing at least one dissolved compound selected from KOH, KHCO₃, and K₂CO₃.

The cathode chamber 11 can receive the CO₂ gas G2 captured in the CO₂ capture part 2. The cathode chamber 11 has a gas flow path facing a non-illustrated reduction electrode, and the CO₂ gas is supplied to the gas flow path. The anode chamber 12 has a liquid flow path facing a non-illustrated oxidation electrode, for example, and the electrolytic solution is supplied to such a liquid flow path. The cathode chamber 11 and the anode chamber 12 are separated by a diaphragm 13 capable of moving at least one ion selected from a hydrogen ion (H⁺), a hydroxide ion (OH⁻), a carbonate ion (CO₃²⁻), and a hydrogen carbonate ion (HCO₃⁻). Examples of the diaphragm 13 include an ion exchange membrane. The CO₂ electrolysis part 3 (the CO₂ electrolysis device having the electrolysis cell) may have a single electrolysis cell or a structure in which electrolysis cells are arranged and connected in a plane direction, or may have a stack structure in which a plurality of the electrolysis cells are stacked to be integrated.

Each of the reduction electrode and the oxidation electrode of the CO₂ electrolysis part 3 is connected to a direct-current power supply 14. The CO₂ electrolysis part 3 can perform the following electrolytic reaction in response to the supply of a direct current from the direct-current power supply 14 to the reduction electrode and the oxidation electrode. That is, the cathode chamber 11 and the anode chamber 12 of the CO₂ electrolysis part 3, can perform the following reactions. The cathode chamber 11 can perform the following electrolytic reaction and the following CO₂ reduction reaction presented by the following formula (1). The cathode chamber 11 can perform the reduction reaction of CO₂ to produce CO and a carbonate ion (CO₃²⁻).

2CO₂ + 2e⁻ → CO + CO₃²⁻ ... (1)

The carbonate ion (CO₃²⁻) produced in the cathode chamber 11 moves to the anode chamber 12 via the diaphragm 13. The anode chamber 12 can perform an oxidation reaction of the carbonate ion (CO₃²⁻), which is produced in the cathode chamber 11 and moved via the diaphragm 13, to produce CO₂ and O₂, as presented in the following formula (2).

CO₃²⁻ → CO₂ + 0.5O₂ + 2e⁻ ... (2)

Moreover, the cathode chamber 11 can perform an electrolytic reaction of H₂O in the electrolytic solution to produce hydrogen (H₂) and a hydroxide ion (OH⁻), as presented in the following formula (3).

2H₂O + 2e⁻ → H₂ + 2OH⁻ ... (3)

The hydroxide ion (OH⁻) produced in the cathode chamber 11 move to the anode chamber 12 via the diaphragm 13. Then, as presented in the following formula (4), water (H₂O) and oxygen (O₂) are produced in the anode chamber 12.

2OH⁻ → 0.5O₂ + H₂O + 2e⁻ ... (4)

Further, in the anode chamber 12, as presented in the following formula (5), water (H₂O) in the electrolytic solution is electrolyzed to produce oxygen (O₂) and a hydrogen ion (H⁺).

2H₂O → 4H⁺ + Q₂ + 4e⁻ ... (5)

The produced hydrogen ion (H⁺) move to the cathode chamber 11 via the diaphragm 13. The hydrogen ion (H⁺) reach the cathode chamber 11, and in the cathode chamber 11 which electrons (e⁻) reach through an external circuit, hydrogen is produced by a reaction presented in the following formula (6).

4H⁺ + 4e⁻ → 2H₂ ... (6)

The cathode chamber 11 can perform the CO₂ reduction reaction presented in the formula (1) to produce CO, and perform the H₂O electrolytic reaction presented in the formula (3) and the reaction presented in the formula (6) to produce H₂. CO and/or H₂ produced in the cathode chamber 11 are/is discharged from the cathode chamber 11 with unreacted CO₂ and saturated steam. A mixed gas (first gas) G3, discharged from the cathode chamber 11, containing CO and/or H₂ and/or CO₂ is subjected to a non-illustrated separation of condensed water through cooling, and thereafter supplied to the organic-substance synthesis part 4 as a part of the feed gas for a synthesis reaction of the organic substance. The cathode chamber 11 is connected to the organic-substance synthesis part 4 via a mixer 15, a CO/H₂ concentration meter 16, a second flowmeter 17, a second buffer tank 18, and a compressor 19. The mixed gas G3 is fed to the second buffer tank 18, and pressurized to a predetermined pressure by the compressor 19, and thereafter fed to the organic-substance synthesis part 4. Although illustration is omitted, a CO₂ separation part which separates and captures CO₂ from the mixed gas G3 may be provided between the cathode chamber 11 and the organic-substance synthesis part 4 (for example, between the cathode chamber 11 and the mixer 15). The CO₂ separated and captured here is fed to the cathode chamber 11 again.

The organic-substance synthesis part 4 received the mixed gas containing CO and/or H₂, can perform at least one reaction selected from a synthesis reaction of an organic substance using, for example, a Fischer-Tropsch synthesis reaction, for example, a reaction of synthesizing hydrocarbon, alcohol, other organic substances, or the like. Examples of the organic substance synthesized in the organic-substance synthesis part 4, include a carbon-containing liquid fuel. The product of the organic-substance synthesis part 4 (organic substance) is discharged from the organic-substance synthesis part 4, and fed to, for example, a separately installed storage facility such as a tank (not illustrated). From the organic-substance synthesis part 4, a synthesis residual gas G4 of the organic substance is discharged. The synthesis residual gas G4 contains an unnecessary product of lower hydrocarbon such as remaining CO, H₂, CO₂, or methane (CH₄), or the like. Such a synthesis residual gas G4 discharged from the organic-substance synthesis part 4 is subjected to an increase in temperature by a regenerative heat exchanger 20, and thereafter fed to a partial oxidation chamber 21 of the oxygen permeation-membrane partial oxidation part 5. The oxygen permeation-membrane partial oxidation part 5 is constituted by the partial oxidation chamber 21, an oxygen removal chamber 22, and an oxygen permeation membrane 23 disposed between them.

Meanwhile, The anode chamber 12 of the CO₂ electrolysis part 3 can perform the oxidation of a carbonate ion (CO₃²⁻) and a hydroxide ion (OH⁻) to produce oxygen (O₂) and carbon dioxide (CO₂), as presented in the formula (2) and the formula (4),. A gas containing the O₂ and CO₂ produced in the anode chamber 12 (O₂-CO₂ containing gas/second gas) is discharged from the anode chamber 12 with the electrolytic solution. The electrolytic solution containing the O₂-CO₂ containing gas is fed to a gas/liquid separator 24, and the O₂-CO₂ containing gas is separated from the electrolytic solution. The electrolytic solution separated by the gas/liquid separator 24 is cooled to a predetermined temperature by a cooler 25, and thereafter returned to the anode chamber 12.

An O₂-CO₂ containing gas G5 separated by the gas/liquid separator 24 contains CO₂ having a relatively high concentration in which a CO₂ concentration is 30 to 60 vol%, and thus emitting the O₂-CO₂ containing gas G5 as it is into the air prevents efficient use of CO₂, and fails to prevent global warming due to CO₂, or the like. Moreover, the O₂-CO₂ containing gas G5 contains a relatively large amount of O₂, and thus feeding the O₂-CO₂ containing gas G5 as it is to the cathode chamber 11 lowers an operation and a function of the CO₂ electrolysis part 3. Therefore, the O₂-CO₂ containing gas G5 discharged from the anode chamber 12 of the CO₂ electrolysis part 3 and separated and captured by the gas/liquid separator 24 is fed to the oxygen removal chamber 22 of the oxygen permeation-membrane partial oxidation part 5. The O₂-CO₂ containing gas G5 is fed to the oxygen removal chamber 22 via a cooler 26, a gas/liquid separator 27, a third flow rate regulating valve 28, and a regenerative heat exchanger 29. The O₂-CO₂ containing gas G5 is cooled to a predetermined temperature by the cooler 26, subjected to a separation of condensed water by the gas/liquid separator 27 and an increase in temperature by the regenerative heat exchanger 29, and thereafter fed to the oxygen removal chamber 22.

The oxygen permeation-membrane partial oxidation part 5 includes the partial oxidation chamber 21, the oxygen removal chamber 22, and the oxygen permeation membrane 23 disposed to separate them. As the oxygen permeation membrane 23, a dense solid oxide electrolyte layer or the like is used. The solid oxide electrolyte layer is an ion conductor passing ions such as oxygen ions, but not passing gas. As the solid oxide electrolyte layer, for example, stabilized zirconia solid-dissolved with an oxide of a rare-earth element such as Y, Sc, Ce, Gd, or Sm used as a stabilizer, typically, yttria-stabilized zirconia (YSZ), ceria-stabilized zirconia (CSZ), or a composite of them is used.

The oxygen permeation-membrane partial oxidation part 5 has a structure in which the partial oxidation chamber 21 as a first electrode chamber having a first electrode and the oxygen removal chamber 22 as a second electrode chamber having a second electrode are separated by the oxygen permeation membrane 23 formed of the solid oxide electrolyte layer or the like. The two electrodes are directly connected to be shorted therebetween, for example. The oxygen removal chamber 22 as the second electrode chamber is responsible for the conversion of oxygen molecules (O₂) into oxygen ions (O²⁻). The partial oxidation chamber 21 as the first electrode chamber is responsible for the conversion of oxygen ions (O²⁻) into oxygen molecules (O₂), and responsible for partial oxidation of the lower hydrocarbon such as methane (CH₄). When a mixed conductor is used as the oxygen permeation membrane 23, the short caused by the direct connection between the electrodes (catalyst layers) is unnecessary. As the mixed conductor, perovskite such as (La₁₋ₓSrₓ)(Co_{1-y}Fe_{y})O₃, (Ba₁₋ₓSrₓ)(Co_{1-y}Fe_{y})O₃, (Pr₁₋ₓSrₓ)(Fe_{1-y}Al_{y})O₃, or (La_{1-x-y}BaₓSr_{y})(Fe_{1-z}In_{z})O₃, (Ce₁₋ₓREₓ)O₂-MnFe₂O₄(RE = La, Pr, Sm, Gd), or a composite of them is used.

In the oxygen permeation-membrane partial oxidation part 5 as described above, the O₂-CO₂ containing gas G5 discharged from the anode chamber 12 of the CO₂ electrolysis part 3 is supplied to the oxygen removal chamber 22. In the oxygen removal chamber 22, O₂ in the O₂-CO₂ containing gas G5 is converted into oxygen ions (O²⁻), as presented in the following formula (7).

O₂ + 4e⁻ - 2O²⁻ ... (7)

The oxygen ions (O²⁻) converted in the oxygen removal chamber 22 are fed to the partial oxidation chamber 21 via the oxygen permeation membrane 23. The oxygen ions (O²⁻) fed to the partial oxidation chamber 21 are converted into an oxygen molecule (O₂), as presented in the following formula (8).

2O²⁻ → O₂ + 4e⁻ ... (8)

The partial oxidation chamber 21 can partially oxidize an oxidizable substance (substance to be oxidized) such as methane (CH₄) contained in the synthesis residual gas G4 to convert the oxidizable substance into CO and H₂, as presented in the following formula (9). The partial oxidation chamber 21 can produce a gas containing CO and H₂ (CO-H₂ containing gas/third gas). The partial oxidation indicates a reaction in which in the oxidation of the lower hydrocarbon such as, for example, methane (CH₄), as presented in the formula (9), without oxidation to stable CO₂, C is partially oxidized to produce H₂-rich CO (partial oxidation state of C).

2CH₄ + O₂ → 2CO + 4H₂ ... (9)

Moreover, as presented in the following formula (10), CO₂ and H₂ are converted into CO and H₂O by a reverse water shift reaction.

CO₂ + H₂ - CO + H₂O ... (10)

The oxygen permeation-membrane partial oxidation part 5 may include an oxygen ion-conducting electrolysis device or an oxygen ion-conducting fuel cell. The oxygen ion-conducting electrolysis device can include, for example, a solid oxide electrolysis cell (SOEC). The SOEC includes a hydrogen electrode chamber, an oxygen electrode chamber, and an oxygen permeation membrane disposed to isolate them. A hydrogen electrode (cathode) disposed in the hydrogen electrode chamber and an oxygen electrode (anode) disposed in the oxygen electrode chamber are connected to a power supply. As the oxygen ion-conducting fuel cell, for example, a solid oxide fuel cell (SOFC) is used. The SOFC includes a fuel electrode chamber, an air electrode chamber, and an oxygen permeation membrane disposed to isolate them.

In this case, the total amount of the O₂-CO₂ containing gas discharged from the anode chamber 12 of the CO₂ electrolysis part 3 is supplied to the reduction electrode of the SOFC or the SOEC. By controlling an oxygen transfer amount from the reduction electrode of the SOFC or the SOEC to the oxidation electrode of the SOFC or the SOEC by using a current value, a CO concentration of a discharge gas from the oxidation electrode of the SOFC or the SOEC may be regulated. The reduction electrode of the SOFC or the SOEC, can perform a reaction presented in the following formula (11). The oxidation electrode of the SOFC or the SOEC, can perform reactions presented in the following formula (12) and formula (13).
Reduction electrode:

   O₂ + 2e⁻ → 2O²⁻ ... (11)
Oxidation electrode:

   2O²⁻ → O₂ + 2e⁻ ... (12)

   CH₄ + O₂ → CO + 2H₂ ... (13)

From the partial oxidation chamber 21 of the oxygen permeation-membrane partial oxidation part 5, a gas G6 containing CO and H₂ (CO-H₂ containing gas (third gas)) is discharged. The CO-H₂ containing gas G6 is used as a part of a feed gas in the organic-substance synthesis part 4, and thus fed to the mixer 15 via the regenerative heat exchanger 20, a cooler 30, a gas/liquid separator 31, and a CO concentration meter 32. The CO-H₂ containing gas G6 is cooled by the regenerative heat exchanger 20 and the cooler 30, subjected to a removal of condensed water by the gas/liquid separator 31, and thereafter fed to the second buffer tank 18 via the mixer 15. The CO-H₂ containing gas G6 is fed from the second buffer tank 18 to the compressor 19 in a mixed state with the CO-H₂ containing gas G3, and pressurized to a predetermined pressure by the compressor 19, and thereafter fed to the organic-substance synthesis part 4.

From the oxygen removal chamber 22 of the oxygen permeation-membrane partial oxidation part 5, an O₂-CO₂ containing gas whose oxygen concentration is lowered (low-concentration O₂-CO₂ containing gas) G7 is discharged. For example, the O₂-CO₂ containing gas may have an oxygen concentration lower than an oxygen concentration of the CO₂ gas G2. The low-concentration O₂-CO₂ containing gas G7 contains CO₂ in a relatively high concentration, and is thus subjected to an increase in temperature by the regenerative heat exchanger 29 to be thereafter returned to the CO₂ capture part 2 for reuse. The low-concentration O₂-CO₂ containing gas G7 contains a certain degree of O₂ despite lowering its oxygen concentration. Thus, the low-concentration O₂-CO₂ containing gas G7 is returned to the CO₂ capture part 2, subjected to separation and capture of CO₂ in the CO₂ capture part 2, and fed to the cathode chamber 11 of the CO₂ electrolysis part 3 as the CO₂ gas G2 whose CO₂ concentration is increased, to be reused. The O₂-CO₂ containing gas G7 having a low O₂ concentration allows, for example, a reduction in oxygen degradation of the amine aqueous solution (amine absorption solution) used for the CO₂ capture part 2.

As described above, feeding the synthesis residual gas G4 containing the unnecessary product such as methane (CH₄), or the like to the partial oxidation chamber 21 of the oxygen permeation-membrane partial oxidation part 5, and partially oxidizing methane (CH₄) or the like to resynthesize CO and H₂ allow efficient use of the synthesis residual gas G4 as a part of the feed gas in the organic-substance synthesis part 4. Further, the supply of the O₂-CO₂ containing gas G5 discharged from the anode chamber 12 of the CO₂ electrolysis part 3 to the oxygen removal chamber 22 contributes to the above-described reproduction of CO and H₂, and thereafter an oxygen concentration in the O₂-CO₂ containing gas G5 is lowered. This allows the return of the low-concentration O₂-CO₂ containing gas G7 whose CO₂ concentration is increased to the CO₂ capture part 2 and a promotion of the reuse of CO₂.

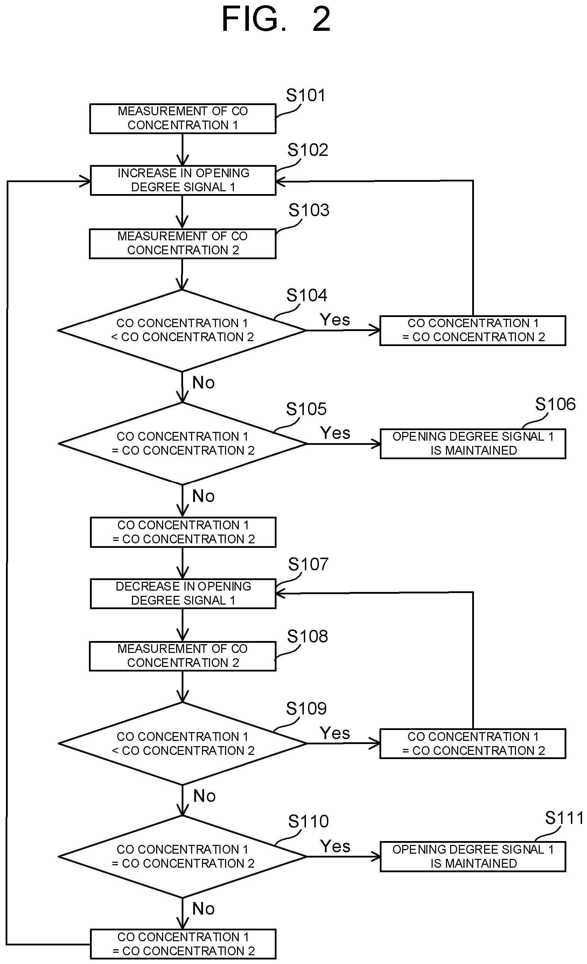

A CO concentration in the CO-H₂ containing gas G6 produced in the partial oxidation chamber 21 of the oxygen permeation-membrane partial oxidation part 5 varies depending on a flow rate of the O₂-CO₂ containing gas G5 supplied to the oxygen removal chamber 22, or the like. Thus, the CO concentration in the CO-H₂ containing gas G6 is measured by the CO concentration meter 32 provided on a downstream side of the partial oxidation chamber 21 and the gas/liquid separator 31, and the flow rate of the O₂-CO₂ containing gas G5 is controlled based on the measured CO concentration by the third flow rate regulating valve 28. This allows the CO concentration in the gas G6 to increase and a CO amount in the feed gas in the organic-substance synthesis part 4 to increase. A concrete flow rate regulation method for the CO concentration in the CO-H₂ containing gas G6 is as illustrated in FIG. 2, for example.

As illustrated in FIG. 2, first, a CO concentration 1 in the CO-H₂ containing gas G6 is measured (S101), and the measurement data of the CO concentration 1 is sent to a first measurement control part 33. Next, an opening degree signal 1 to increase an opening degree of the third flow rate regulating valve 28 is sent from the first measurement control part 33 to the third flow rate regulating valve 28 (S102). After increasing the opening degree of the third flow rate regulating valve 28, a CO concentration 2 in the gas G6 is measured (S103), and whether the CO concentration 2 is higher than the CO concentration 1 (CO concentration 1 < CO concentration 2) is determined (S104). When the CO concentration 2 is higher than the CO concentration 1 (yes), the opening degree signal 1 of the third flow rate regulating valve 28 is increased (S102).

Similarly, the measurement of the CO concentration 2 in the gas G6 (S103) and the determination of whether the CO concentration 2 is higher than the CO concentration 1 (S104) are performed. When the CO concentration 2 is higher than the CO concentration 1 (yes), the above-described steps are performed, and when the CO concentration 2 is not higher than the CO concentration 1 (no), whether the CO concentration 2 is roughly equal to the CO concentration 1 (CO concentration 1 = CO concentration 2) is determined (S105). When the CO concentration 2 is roughly equal to the CO concentration 1 (yes), the opening degree signal 1 of the third flow rate regulating valve 28 is maintained (S106). When the CO concentration 2 is not roughly equal to the CO concentration 1 (no), the opening degree signal 1 to decrease the opening degree of the third flow rate regulating valve 28 is sent from the first measurement control part 33 to the third flow rate regulating valve 28 (S107).

Next, the CO concentration 2 in the gas G6 is measured (S108), and whether the CO concentration 2 is higher than the CO concentration 1 (CO concentration 1 < CO concentration 2) is determined (S109). When the CO concentration 2 is higher than the CO concentration 1 (yes), the opening degree signal 1 of the third flow rate regulating valve 28 is decreased (S107). Similarly, the measurement of the CO concentration 2 in the gas G6 (S108) and the determination of whether the CO concentration 2 is higher than the CO concentration 1 (S109) are performed. When the CO concentration 2 is higher than the CO concentration 1 (yes), the above-described steps are performed, and when the CO concentration 2 is not higher than the CO concentration 1 (no), whether the CO concentration 2 is roughly equal to the CO concentration 1 (CO concentration 1 = CO concentration 2) is determined (S110). When the CO concentration 2 is roughly equal to the CO concentration 1 (yes), the opening degree signal 1 of the third flow rate regulating valve 28 is maintained (S111). When the CO concentration 2 is not roughly equal to the CO concentration 1 (no), the opening degree signal 1 to increase the opening degree of the third flow rate regulating valve 28 is sent from the first measurement control part 33 to the third flow rate regulating valve 28 (S102). This manner increases the CO concentration in the gas G7, thereby allowing an increase in the CO amount in the feed gas in the organic-substance synthesis part 4.

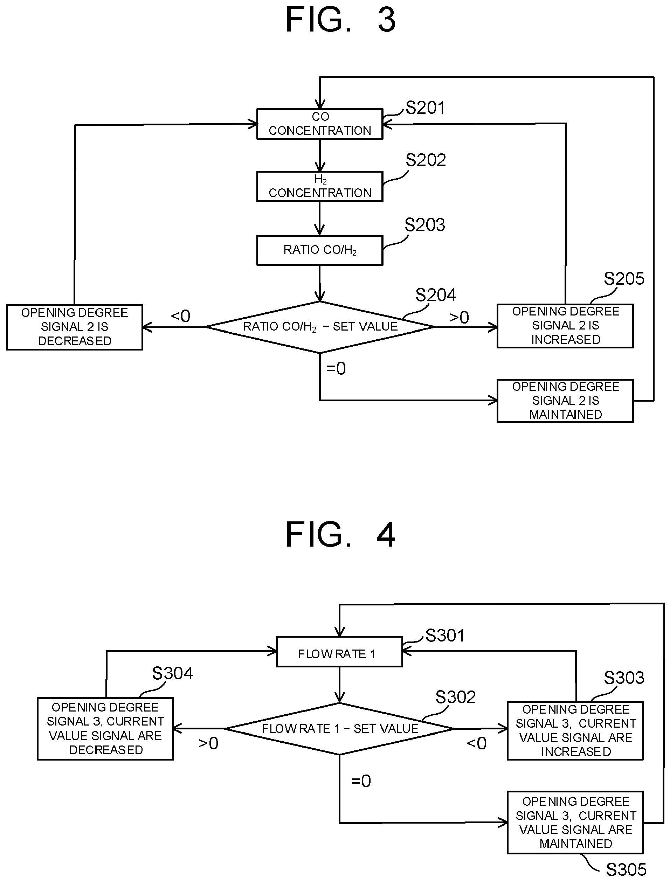

In the CO-H₂ containing gas supplied to the organic-substance synthesis part 4 as the feed gas, increasing the CO concentration in the CO-H₂ containing gas G6 produced in the partial oxidation chamber 21 as described above causes the possibility of an insufficient H₂ amount in the feed gas and a decrease in CO/H₂ ratio. Thus, a supply part of H₂ (H₂ source) 35 is connected to the mixer 15 via a fourth flow rate regulating valve 34. Supplying H₂ from the H₂ source 35 to the CO-H₂ containing gas allows the regulation of the CO/H₂ ratio of the CO-H₂ containing gas to be supplied to the organic-substance synthesis part 4 as the feed gas. However, even though H₂ is uniformly added from the H₂ source 35 to the CO-H₂ containing gas, a set value of the CO/H₂ ratio cannot be maintained. Thus, the CO concentration and the H₂ concentration are measured by the CO/H₂ concentration meter 16 provided on a downstream side of the second buffer tank 18, and based on the measured CO concentration and H₂ concentration, a H₂ addition amount from the H₂ source 35 is controlled by the fourth flow rate regulating valve 34. This allows the CO/H₂ ratio of the CO-H₂ containing gas to approximate the set value, and a yield and synthesis efficiency of the organic-substance synthesis part 4 to increase. A concrete flow rate regulation method for the CO/H₂ ratio in the CO-H₂ containing gas is as illustrated in FIG. 3, for example.

As illustrated in FIG. 3, first, the measurement of the CO concentration (S201) and the measurement of the H₂ concentration (S202) in the CO-H₂ containing gas are performed, and their measurement data is sent to a second measurement control part 36. In the second measurement control part 36, the CO/H₂ ratio is found from the measurement data (S203). Moreover, a value of [CO/H₂ ratio - set value] is found, and whether the value of [CO/H₂ ratio - set value] exceeds 0 is determined (S204). When the value of [CO/H₂ ratio - set value] exceeds 0, an opening degree signal 2 of the fourth flow rate regulating valve 34 is increased (S205). When the value of [CO/H₂ ratio - set value] is less than 0, the opening degree signal 2 of the fourth flow rate regulating valve 34 is decreased (S205). When the value of [CO/H₂ ratio - set value] is 0, the opening degree signal 2 of the fourth flow rate regulating valve 34 is maintained. Repeating such steps allows the CO/H₂ ratio of the CO-H₂ containing gas to approximate the set value.

A flow rate of the CO-H₂ containing gas supplied to the organic-substance synthesis part 4 as the feed gas is also affected by a flow rate of the CO-H₂ containing gas G3 produced in the cathode chamber 11. The yield, the synthesis efficiency, and the like of the organic substance in the organic-substance synthesis part 4 are also affected by a supply amount of the feed gas. Thus, the flow rate of the CO-H₂ containing gas is measured by the second flowmeter 17 provided on an upstream side of the second buffer tank 18, and based on the measured flow rate of the gas, a flow rate of the CO₂ gas G2 to be supplied to the cathode chamber 11 is controlled, and a current value to be supplied from the direct-current power supply 14 to the CO₂ electrolysis part 3 is controlled. This allows the supply amount of the feed gas supplied to the organic-substance synthesis part 4 to approximate a set value. A concrete flow rate regulation method of the flow rate of the CO-H₂ containing gas G3 is as illustrated in FIG. 4, for example.

As illustrated in FIG. 4, first, a flow rate 1 of the CO-H₂ containing gas to be supplied to the organic-substance synthesis part 4 is measured by the second flowmeter 17 (S301), and this measurement data is sent to a third measurement control part 37. In the third measurement control part 37, a value of [flow rate 1 - set value] is found from the measurement data, and whether the value of [flow rate 1 - set value] exceeds 0 is determined (S302). When the value of [flow rate 1 - set value] exceeds 0, an opening degree signal 3 of the second flow rate regulating valve 10 and a signal of a current value (a current value signal) to be supplied from the direct-current power supply 14 are increased (S303). When the value of [flow rate 1 - set value] is less than 0, the opening degree signal 3 of the second flow rate regulating valve 10 and the signal of the current value to be supplied from the direct-current power supply 14 are decreased (S304). When the value of [flow rate 1 - set value] is 0, the opening degree signal 3 of the second flow rate regulating valve 10 and the signal of the current value to be supplied from the direct-current power supply 14 are maintained. Repeating such steps allows the flow rate 1 of the CO-H₂ containing gas to approximate the set value.

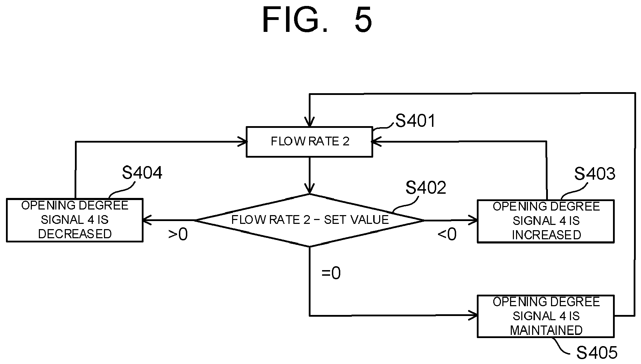

Moreover, the flow rate of the CO-H₂ containing gas supplied to the organic-substance synthesis part 4 as the feed gas is also affected by the flow rate of the CO₂ gas G2 supplied to the cathode chamber 11. Thus, the flow rate of the CO₂ gas G2 is measured by the first flowmeter 8 provided on an upstream side of the first buffer tank 9, and based on the measured flow rate of the gas, a flow rate of the CO₂ containing gas G1 to be supplied to the CO₂ capture part 2 is controlled by the first flow rate regulating valve 6. This allows the supply amount of the feed gas supplied to the organic-substance synthesis part 4 to approximate the set value. A concrete flow rate regulation method of the flow rate of the CO₂ containing gas G1 is as illustrated in FIG. 5, for example.

As illustrated in FIG. 5, first, a flow rate 2 of the CO₂ gas G2 to be supplied to the cathode chamber 11 is measured by the first flowmeter 8 (S401), and this measurement data is sent to a fourth measurement control part 38. In the fourth measurement control part 38, a value of [flow rate 2 - set value] is found from the measurement data, and whether the value of [flow rate 2 - set value] exceeds 0 is determined (S402). When the value of [flow rate 2 - set value] exceeds 0, an opening degree signal 4 of the first flow rate regulating valve 6 is increased (S403). When the value of [flow rate 2 - set value] is less than 0, the opening degree signal 4 of the first flow rate regulating valve 6 is decreased (S404). When the value of [flow rate 2 - set value] is 0, the opening degree signal 4 of the first flow rate regulating valve 6 is maintained. Repeating such steps allows the flow rate 2 of the CO₂ gas G2 supplied to the cathode chamber 11 to approximate the set value.

In addition to the regulation of the gas concentration, the gas flow rate, and the like as described above, a methane flow rate may be calculated from a flow rate and a methane (CH₄) concentration of the synthesis residual gas G4 of the organic substance discharged from the organic-substance synthesis part 4, to control the flow rate of the O₂-CO₂ containing gas G5 discharged from the anode chamber 12 based on the methane flow rate. In a required flow rate of the O₂-CO₂ containing gas G5, a correction of a water vapor content by using a gas temperature or a correction using by an oxygen concentration is also effective. A required oxygen amount can be calculated using characteristics of the oxygen permeation-membrane partial oxidation part 5. Moreover, from a flow rate, a CO concentration, and a H₂ concentration after mixing the CO and H₂ containing gas G3 discharged from the cathode chamber 11 of the CO₂ electrolysis part 3 and the CO and H₂ containing gas G6 discharged from the partial oxidation part 21 of the oxygen permeation-membrane partial oxidation part 5, a hydrogen flow rate required for a predetermined CO/H₂ ratio may be calculated to supply hydrogen.

From a flow rate and a CO₂ concentration after mixing a bypass flow of the O₂-CO₂ containing gas discharged from the anode chamber 12 of the CO₂ electrolysis part 3 and the low-concentration O₂-CO₂ containing gas discharged from the oxygen removal chamber of the oxygen permeation-membrane partial oxidation part 5, and a CO₂ concentration of the CO₂ containing gas, a supply amount of the CO₂ containing gas may be controlled.

Note that though not described and illustrated in the above-described embodiment and FIG. 1, a blower, a pump, a compressor, or the like may be appropriately disposed, and the flow of the fluid may be assisted by them. Further, a part of a discharge gas from the CO₂ capture part may be supplied for other uses such as underground storage, EOR, and CO₂ fixation (mineralization).

Note that the configurations of the above-described embodiments are applicable in combination with each other, and parts thereof are also replaceable. While certain embodiments of the present invention have been described above, these embodiments have been presented by way of example only, and are not intended to limit the scope of the invention. Indeed these embodiments described herein may be embodied in a variety of other forms; furthermore, various omissions, substitutions, and changes in the form of the embodiments described herein may be made without departing from the spirit of the inventions. The accompanying claims and their equivalents are intended to cover such forms or modifications as would fall within the scope and spirit of the inventions.

## Claims

1. A carbon dioxide conversion apparatus comprising:
a carbon dioxide capture part configured to capture a carbon dioxide from a carbon dioxide containing gas;
a carbon dioxide electrolysis part having
a cathode chamber configured to receive the carbon dioxide from the carbon dioxide capture part, and configured to reduce at least one selected from the group consisting of the carbon dioxide and water and produce a first gas containing at least one selected from the group consisting of carbon monoxide and hydrogen, and
an anode chamber configured to oxidize an oxidizable substance and produce a second gas containing oxygen and carbon dioxide;
a direct-current power supply configured to supply a direct current to the carbon dioxide electrolysis part;
an organic-substance synthesis part configured to receive the first gas from the cathode chamber, and configured to synthesize an organic substance from a feed gas containing the first gas;
a partial oxidation part having
an oxygen removal chamber configured to receive the second gas from the anode chamber, and configured to ionize oxygen in the second gas to produce an oxygen ion,
a partial oxidation chamber configured to:
receive a synthesis residual gas to be discharged from the organic-substance synthesis part, the synthesis residual gas containing the organic substance;
partially oxidize the synthesis residual gas using the oxygen ion to be produced in the oxygen removal chamber and produce a third gas containing carbon monoxide and hydrogen; and
supply the third gas as a part of the feed gas to the organic-substance synthesis part, and
an oxygen permeation membrane provided between the oxygen removal chamber and the partial oxidation chamber; and
a first control part configured to control an amount of the second gas to be supplied from the anode chamber to the oxygen removal chamber to increase an amount of carbon monoxide in the third gas to be produced in the partial oxidation chamber.

2. The apparatus according to claim 1, further comprising:
a hydrogen supply part configured to supply a hydrogen gas to the feed gas; and
a second control part configured to control an amount of the hydrogen gas to be supplied from the hydrogen supply part to control a ratio of carbon monoxide and hydrogen in the feed gas.

3. The apparatus according to claim 1, further comprising
a third control part configured to control an amount of carbon dioxide to be supplied to the cathode chamber and a value of the direct current to be supplied from the direct-current power supply to the carbon dioxide electrolysis part to control an amount of the feed gas to be supplied to the organic-substance synthesis part.

4. The apparatus according to claim 1, further comprising
a fourth control part configured to control an amount of the carbon dioxide containing gas to the carbon dioxide capture part to control an amount of carbon dioxide to be supplied from the carbon dioxide capture part to the cathode chamber.

5. The apparatus according to claim 1, wherein
the partial oxidation part is configured to discharge a carbon dioxide-containing residual gas, and supply the carbon dioxide-containing residual gas to the carbon dioxide capture part, the carbon dioxide-containing residual gas having an oxygen concentration lower than an oxygen concentration of the second gas.

6. The apparatus according to claim 1, wherein
the partial oxidation part includes an oxygen ion-conducting electrolysis device.

7. The apparatus according to claim 1, wherein
the partial oxidation part includes an oxygen ion-conducting fuel cell.
